(19) Europäisches Patentamt
European Patent Office
Office européen des brevets

(11) Veröffentlichungsnummer : **0 057 772**
**B1**

(12) **EUROPÄISCHE PATENTSCHRIFT**

(45) Veröffentlichungstag der Patentschrift : **13.03.85**

(51) Int. Cl.[4] : **C 07 C119/045**, C 07 C118/00

(21) Anmeldenummer : **81110722.6**

(22) Anmeldetag : **23.12.81**

(54) **Halogenierte, tertiäre Diisocyanate und Verfahren zu ihrer Herstellung.**

(30) Priorität : **23.01.81 DE 3102089**

(43) Veröffentlichungstag der Anmeldung : **18.08.82 Patentblatt 82/33**

(45) Bekanntmachung des Hinweises auf die Patenterteilung : **13.03.85 Patentblatt 85/11**

(84) Benannte Vertragsstaaten : **BE CH DE FR GB LI**

(56) Entgegenhaltungen :
**EP-A- 0 025 907**
**DE-A- 2 127 262**
**DE-B- 1 122 058**

(73) Patentinhaber : **BASF Aktiengesellschaft**
**Carl-Bosch-Strasse 38**
**D-6700 Ludwigshafen (DE)**

(72) Erfinder : **Koenig, Karl-Heinz, Dr.**
**Pierstrasse 8 A**
**D-6710 Frankenthal (DE)**
Erfinder : **Schwendemann, Volker, Dr.**
**Haupstrasse 64 A**
**D-6901 Wiesenbach (DE)**

**EP 0 057 772 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft halogenierte, tertiäre Diisocyanate und Verfahren zu ihrer Herstellung durch Umsetzung von tertiären Diisocyanaten mit Halogen, Sulfurylchlorid und/oder Sulfurylbromid.

Die Halogenierung von aliphatischen Isocyanaten mit mindestens einem substituierbaren Wasserstoffatom am α-ständigen Kohlenstoff zum Stickstoff der Isocyanatgruppe ist bekannt (DE-PS 11 22 058, Angew. Chemie, Band 74, S. 848 bis 855 (1962) und Band 80, Seiten 942 bis 953 (1968)). In DE-PS 11 22 058 wird darauf hingewiesen, daß Halogenalkylisocyanate sehr reaktionsfähige Substanzen sind (2. Spalte, Zeilen 33 und 34) und α-Halogenalkylisocyanate zur Kondensation oder Polymerisation neigen (2. Spalte, Zeilen 30 und 31) ; Beispiel 3 zeigt neben einer 68 %igen Ausbeute größere Destillationsrückstände.

Ohne Zusatz von Lösungsmitteln treten bei der Halogenierung von aliphatischen Isocyanaten durch intramolekulare Kondensation oft Verharzung und schlechte Ausbeuten auf, wie die Angew. Chemie (loc. cit., Seite 946) lehrt. Ebenfalls zeigt diese Literaturstelle, daß durch Anlagerung des bei der Reaktion gebildeten Chlorwasserstoffs an das Isocyanat große Mengen an dem entsprechenden Carbamidsäurechlorid gebildet werden, und empfiehlt zur Vermeidung dieser Nebenreaktion, daß als Ausgangsstoffe Carbamidsäurechloride verwendet werden.

Führt man das in DE-PS 11 22 058 beschriebene Verfahren der α-Halogenierung aliphatischer Isocyanate im industriellen Maßstab durch, so zeigt sich, daß bei der Aufdestillation des anfallenden Gemisches von halogenierten Isocyanaten verschiedenen Halogenierungsgrades erhebliche, nicht destillierbare Rückstande von Polymeren oder Polykondensaten verbleiben, im allgemeinen ca. 20 bis 40 Gew. %, bezogen auf das halogenierte Produkt.

Es wurde nun gefunden, daß man halogenierte, tertiäre Diisocyanate der Formel I

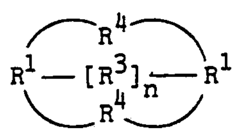

(I)

worin n 0 oder 1 bezeichnet und die einzelnen Reste $R^1$ gleich oder verschieden sind und, wenn n = 0 bedeutet, für den Rest

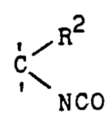

oder den Rest

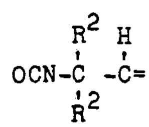

stehen oder, wenn n 1 bedeutet, den Rest

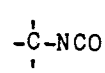

oder den Rest

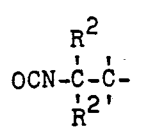

bezeichnen, die einzelnen Reste $R^2$ gleich oder verschieden sind und einen aliphatischen Rest bedeuten, jeweils zwei über ein Kohlenstoffatom benachbarte Reste $R^2$ auch Glieder eines halogenfreien oder halogenierten, alicyclischen Ringes bilden können, die einzelnen Reste $R^3$ und $R^4$ gleich oder verschieden sein können und jeweils einen aliphatischen Rest bezeichnen, und insgesamt der Endstoff mehr Halogenatome als der Ausgangsstoff II besitzt, durch Halogenierung von Isocyanaten vorteilhaft erhält, wenn man tertiäre Diisocyanate der Formel II

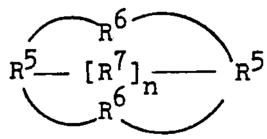 (II)

worin $R^5$ der Bedeutung von $R^1$, $R^6$ der Bedeutung von $R^4$ und $R^7$ der Bedeutung von $R^3$ entspricht, wobei die vorgenannten Reste insgesamt halogenärmer als die Reste $R^1$, $R^4$, $R^3$ oder halogenfrei sind, mit Halogen, Sulfurylchlorid und/oder Sulfurylbromid umsetzt.

Weiterhin wurden die neuen halogenierten, tertiären Diisocyanate der Formel I

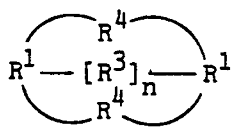 (I)

worin n 0 oder 1 bezeichnet und die einzelnen Reste $R^1$ gleich oder verschieden sind und, wenn n = 0 bedeutet, für den Rest

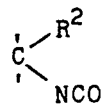

oder den Rest

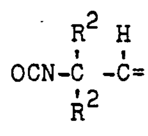

stehen oder, wenn n 1 bedeutet, den Rest

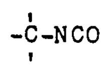

oder den Rest

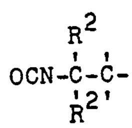

bezeichnen, die einzelnen Reste $R^2$ gleich oder verschieden sind und einen aliphatischen Rest bedeuten, jeweils zwei über ein Kohlenstoffatom benachbarte Reste $R^2$ auch Glieder eines halogenfreien oder halogenierten alicyclischen Ringes bilden können, die einzelnen Reste $R^3$ und $R^4$ gleich oder unterschiedlich sein können und jeweils einen aliphatischen Rest bezeichnen, gefunden.

Die Umsetzung wird für den Fall der Verwendung von p-Menthandiisocyanat und Chlor durch die folgenden Formeln wiedergegeben :

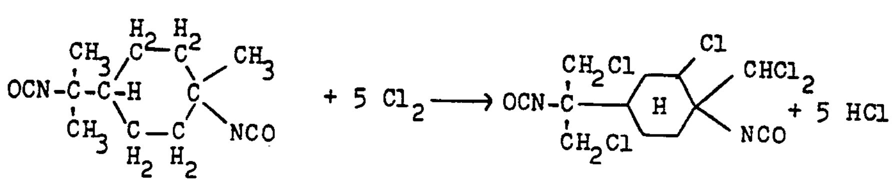


Im Hinblick auf den Stand der Technik liefert das Verfahren nach der Erfindung überraschend auf einfachem und wirtschaftlichem Wege halogenierte, tertiäre Diisocyanate in guter Ausbeute und Reinheit. Es was überraschend, daß gerade kein heterogenes Gemisch zahlreicher Chlorierungsstufen, sondern definierte Stoffe entstehen und keine oder nur geringfügige Destillationsrückstände sich ergeben.

Bevorzugte Ausgangsstoffe II und demzufolge bevorzugte Endstoffe I sind solche, in deren Formeln n

0 oder 1 bezeichnet, die einzelnen Reste $R^1$ gleich oder verschieden sein können und, wenn n = 0 bedeutet, für den Rest

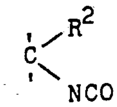

oder den Rest

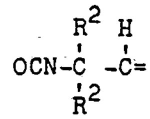

stehen, oder, wenn n 1 bedeutet, den Rest

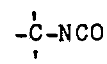

oder den Rest

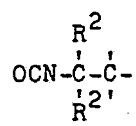

bezeichnen, die einzelnen Reste $R^2$ gleich oder verschieden sind und einen halogenfreien oder halogenierten Alkylrest mit 1 bis 6 Kohlenstoffatomen bedeuten, jeweils zwei über ein Kohlenstoffatom benachbarte Reste $R^2$ auch Glieder eines halogenfreien oder halogenierten alicyclischen 5- bis 7-gliedrigen Ringes bilden können, die einzelnen Reste $R^3$ und $R^4$ gleich oder verschieden sein können und jeweils einen halogenfreien oder halogenierten Alkylenrest mit 1 bis 4 Kohlenstoffatomen bezeichnen. Zweckmäßig ist der Ausgangsstoff II halogenfrei; gegebenenfalls kann er schon 1 bis 3 Halogenatome tragen. $R^5$ entspricht in seiner Bedeutung $R^1$, $R^6$ der Bedeutung von $R^4$ und $R^7$ der Bedeutung von $R^3$ und jeder Rest unterscheidet sich gegebenenfalls von den Resten $R^3$, $R^1$ und $R^4$ durch Halogenfreiheit oder einen niedrigeren Halogenierungsgrad. Zweckmäßig enthält der Endstoff I 1 bis 10, insbesondere 3 bis 7 Halogenatome mehr als der Ausgangsstoff II. Im allgemeinen trägt mindestens einer der Reste $R^2$ ein oder mehrere Halogenatome. Unter den Halogenen sind Brom und insbesondere Chlor bevorzugt.

Beispielsweise sind folgende tertiäre Diisocyanate II geeignet: 1,4-Dimethyl-cyclohexan-1,4-diisocyanat und die entsprechenden 1,4-Diäthyl-, 1,4-Dipropyl-, 1,4-Diisopropyl-, 1,4-Dibutyl-, 1,4-Di-sek.butyl-, 1,4-Diisobutyl-, 1,4-Di-tert.-butylderivate; analog substituierte 1,3- und 1,2-Cyclohexandiisocyanate; p-Methan-1,4-diisocyanat sowie die homologen meta-Methan-1,3-diisocyanate und o-Menthan-1,2-diisocyanate; homologe Verbindungen, deren 3-Methylgruppen gleich oder unterschiedlich durch die Äthyl-, Propyl-, Isopropyl-, Butyl-, Isobutyl-, sek.-Butyl-, tert.-Butylgruppe ersetzt sind; Dicyclo-[2,2,2]-hexan-1,4-diisocyanat und entsprechende [3,3,3]-Nonane, [2,1,2]-hexan-Derivate; durch die Methyl-, Äthyl-, Propyl-, Isopropyl-, Butyl-, sek.-Butyl-, tert.-Butyl- oder Isobutyl-Gruppe und Isocyanatgruppe in 1,2- und 1,3-Stellung substituierten Cyclopentane und in 1,2-, 1,3-, 1,4-Stellung substituierte Cycloheptane; bevorzugt sind p-Menthan-1,4-diisocyanat, 1,4-Dimethylcyclohexyl-1,4-diisocyanat.

Man setzt sie Ausgangsstoffe II mit Halogen, Sulfurylchlorid oder Sulfurylbromid als Halogenierungsmittel, bevorzugt mit Jod, Brom und insbesondere mit Chlor, um. Im allgemeinen erhält man bei der Halogenierung Gemische halogenierter Endstoffe I mit unterschiedlicher Zahl der Halogensubstituenten. Zuerst werden bevorzugt die β-ständigen Kohlenstoffatome halogeniert. Die Hauptkomponente an den Endstoffen I im Gemisch ist in der Regel eine dem Molverhältnis von Ausgangsstoff und Halogenierungsmittel entsprechende Zahl der Halogensubstituenten am Molekül.

Die Ausgangsstoffe II können in stöchiometrischer Menge verwendet werden. Bevorzugt wird man für die Herstellung von disubstituierten, trisubstituierten und höher halogenierten Alkylisocyanaten einen Überschuß an Halogenierungsmittel, bezogen auf Ausgangsstoff II, verwenden; es kommen Mengen über der stöchiometrischen Menge bis zu 15 Mol, zweckmäßig von 0,5 bis 8 Mol Halogenierungsmittel, bezogen auf Mol Ausgangsstoff II, in Betracht. Bei der Herstellung von weitgehend monosubstituierten Verbindungen wird vorteilhaft ein Unterschuß an Chlor (Anchlorierung) verwendet, es kommen Mengen unterhalb der stöchiometrischen Menge bis zu 0,95 Mol, zweckmäßig von 0,6 bis 0,9 Mol, bezogen auf Ausgangsstoff II, Halogenierungsmittel in Frage. Bevorzugt wird für höherhalogenierte Produkte eine

Kreislaufhalogenierung verwendet, bei der nicht verbrauchtes elementares Halogen wieder zurückgeführt werden kann. Im übrigen kann der Halogenüberschuß umso geringer sein, je schneller das Halogen umgesetzt wird. Bei der Verwendung von Sulfurylhalogeniden setzt man zweckmäßig einen Halogenierungskatalysator wie Benzoylperoxid, Azodiisobutyronitril oder Ascaridol in katalytischen Mengen, zweckmäßig von 0,01 bis 0,1 Gew.%, bezogen auf das Halogenierungsmittel, zu.

Die Reaktion wird bevorzugt unter Belichtung durchgeführt, wobei für die Erzeugung des Lichtes vorzugsweise alle im sichtbaren Bereich strahlenden Lichtquellen verwendet werden können. So kann z. B. mit Sonnenlicht oder künstlichem Licht, z. B. von Quarzbrennern, Quecksilberdampflampen, Tageslichtlampen, Leuchtstoffröhren, belichtet werden. Vorteilhaft verwendet man Tauchlampen, die vom Reaktionsgemisch umspült werden, insbesondere im Falle von Kreislaufapparaten in einem der Vertikalrohre. Bevorzugt sind Lichtquellen mit einem hohen Strahlungsanteil im Bereich der Wellenlängen von 3 000 bis 5 000 Angström.

Die Halogenierungstemperatur richtet sich vorteilhaft nach dem Halogenierungsgrad des Endstoffs I. So ist es bei niedrigen gewünschten Halogenierungsgraden, z. B. der Substituierung der Ausgangsstoffe II mit 1 bis 2 Halogenatomen, zweckmäßig, zunächst bei einer Temperatur von 0 bis 10 °C zu beginnen und die Temperatur langsam bis ca. 50 °C zu steigern. Bei höheren erwünschten Halogenierungsgraden, z. B. die Substituierung der Ausgangsstoffe II mit 3 bis 6 Halogenatomen beginnt man zur schnelleren Halogenaufnahme schon bei Temperaturen von 50 bis 100 °C, und steigert die Temperatur rascher. Mit fortschreitendem Halogenierungsgrad ist es zur Aufrechterhaltung einer hinreichenden Halogenaufnahme vorteilhaft, die Reaktionstemperatur bis maximal 230 °C, bevorzugt 160 bis 180 °C zu steigern. In der Regel wird die Umsetzung bei einer Temperatur von – 10 bis 230 °C, vorzugsweise 40 bis 180 °C, insbesondere 60 bis 130 °C, drucklos oder unter Druck, kontinuierlich oder diskontinuierlich durchgeführt. Bevorzugt wird in Abwesenheit zusätzlicher Lösungsmittel umgesetzt.

Die Reaktion kann wie folgt durchgeführt werden :

Der Ausgangsstoff II wird bei der Reaktionstemperatur mit dem Halogenierungsmittel während 2 bis 50 Std. umgesetzt, wobei im Falle von elementarem Halogen dieses langsam und portionsweise während der Reaktionszeit zugesetzt wird. Zweckmäßig wird in vorgenannter Weise die Temperatur innerhalb des vorgenannten Temperaturintervalls während der Reaktion erhöht. Aus dem Reaktionsgemisch wird im Falle eines einzelnen Hauptprodukts der Endstoff in üblicher Weise, z. B. durch fraktionierte Destillation, abgetrennt. In der Mehrzahl der Fälle, gerade im industriellen Betrieb, handelt es sich um Gemische von Endstoffen, die durch Destillation gereinigt werden können, meistens aber der Weiterverarbeitung, z. B. der Herstellung von appretiermitteln, z. B. Flammschutzmitteln, direkt zugeführt werden. Die niederhalogenierten Isocyanate des Destillationsvorlaufs kann man wieder als Vorprodukte für die Synthese höherhalogenierter Fraktionen eines nächsten Ansatzes verwenden. Die maximale Halogenaufnahme wird bei etwa 80 bis 85 % der Theorie erreicht.

Die nach dem Verfahren der Erfindung herstellbaren, halogenierten, tertiären Diisocyanate sind wertvolle Ausgangsstoffe für die Herstellung von Schädlingsbekämpfungsmitteln, Lederfettungsmitteln, Pharmaceutica, Flammschutzmitteln, Schmierölen, Kunstharzen, Gleitmitteln. So lassen sich die mehrfach chlorierten Diisocyanate direkt als bifunktionelle Synthesebausteine für flammfeste Polymere, z. B. Polyurethane, verwenden. Bezüglich der Verwendung wird auf vorgenannte Veröffentlichungen verwiesen.

Die in den folgenden Beispielen aufgeführten Teile bedeuten Gewichtsteile.

## Beispiel

Chlorierung von p-Methandiisocyanat

222 Teile p-Menthandiisocyanat ($Kp_{0,1\ mbar}$ 86-8° ; $n_D^{24}$ = 1,472 0) werden in 500 Teilen Tetrachlorkohlenstoff gelöst und in einer Umlaufchlorierungsapparatur, die mit einer Labortauchlampe versehen ist, bei Raumtemperatur mit Chlorgas begast. Die Reaktion ist exotherm ; man begast, bis bei Siedetemperatur des Tetrachlorkohlenstoff kein Chlor mehr aufgenommen wird. Dann zieht man das Lösungsmittel im Vakuum ab und chloriert das farblos bis hellgelbe Öl bei 120 °C weiter bis zur Aufnahme von 115 Teilen Chlor. Das Rohprodukt hat einen Brechungsindex von $n_D^{21}$ = 1,526 0. Bei der groben fraktionierten Destillation erhält man folgende Fraktionen :

$Kp_{(0,4\ mbar)}$ 125- 50°- 7 Teile (Vorlauf) $n_D^{21}$ = 1,514 0
$Kp_{(0,4\ mbar)}$ 157- 64°-130 Teile (Vorlauf) $n_D^{21}$ = 1,516 0
$Kp_{(0,4\ mbar)}$ 165-175°-110 Teile (Vorlauf) $n_D^{21}$ = 1,522 5
$Kp_{(0,4\ mbar)}$ 175-184°- 59 Teile (Vorlauf) $n_D^{21}$ = 1,530 0

Die Fraktionen 2 und 3 enthalten 2 bis 7 Chloratome pro Molekül Isocyanat.

**Ansprüche**

1. Verfahren zur Herstellung von halogenierten, tertiären Diisocyanaten der Formel I

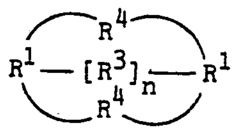

(I)

worin n 0 oder 1 bezeichnet und die einzelnenen Reste $R^1$ gleich oder verschieden sind und, wenn n = 0 bedeutet, für den Rest

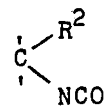

oder den Rest

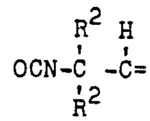

stehen oder, wenn n 1 bedeutet, den Rest

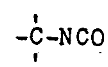

oder den Rest

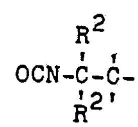

bezeichnen, die einzelnen Reste $R^2$ gleich oder verschieden sind und einen aliphatischen Rest bedeuten, jeweils zwei über ein Kohlenstoffatom benachbarte Reste $R^2$ auch Glieder eines halogenfreien oder halogenierten, alicyclischen Ringes bilden können, die einzelnen Reste $R^3$ und $R^4$ gleich oder verschieden sein können und jeweils einen aliphatischen Rest bezeichnen, und insgesamt der Endstoff mehr Halogenatome als der Ausgangsstoff II besitzt, durch Halogenierung von Isocyanaten, dadurch gekennzeichnet, daß man tertiäre Diisocyanate der Formel II

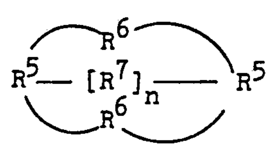

(II)

worin $R^5$ der Bedeutung von $R^1$, $R^6$ der Bedeutung von $R^4$ und $R^7$ der Bedeutung von $R^3$ entspricht, wobei die vorgenannten Reste insgesamt halogenärmer als die Reste $R^1$, $R^4$ und $R^3$ oder halogenfrei sind, mit Halogen, Sulfurylchlorid und/oder Sulfurylbromid umsetzt.

2. Halogenierte, tertiäre Diisocyanate der Formel I

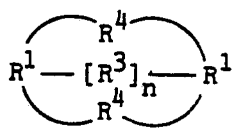

(I)

worin n 0 oder 1 bezeichnet und die einzelnen Reste $R^1$ gleich oder verschieden sind und, wenn n = 0 bedeutet, für den Rest

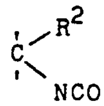

oder den Rest

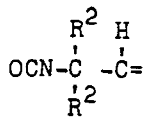

stehen oder, wenn n 1 bedeutet, den Rest

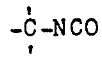

oder den Rest

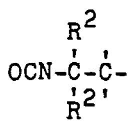

bezeichnen, die einzelnen Reste $R^2$ gleich oder verschieden sind und einen aliphatischen Rest bedeuten, jeweils zwei über ein Kohlenstoffatom benachbarte Reste $R^2$ auch Glieder eines halogenfreien oder halogenierten, alicyclischen Ringes bilden können, die einzelnen Reste $R^3$ und $R^4$ gleich oder verschieden sein können und jeweils einen aliphatischen Rest bezeichnen.

**Claims**

1. A process for the preparation of a halogenated, tertiary diisocyanate of the formula I

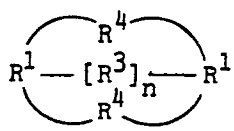

(I)

where n is 0 or 1 and the individual radicals $R^1$ are identical or different and, if n is 0, are

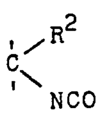

or

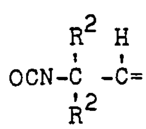

or, if n is 1, are 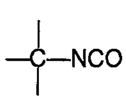

or

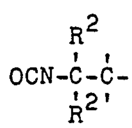

the individual radicals $R^2$ are identical or different and are aliphatic radicals, or two adjacent radicals $R^2$ linked by a carbon atom can be members of a halogen-free or halogenated alicyclic ring, and the individual radicals $R^3$ and $R^4$ can be identical or different and are aliphatic radicals, the end product

having more halogen atoms than the starting material II, by halogenation of an isocyanate, wherein a tertiary diisocyanate of the formula II

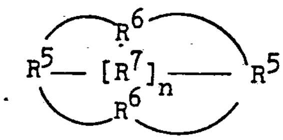

(II)

where $R^5$ has the meanings of $R^1$, $R^6$ has the meanings of $R^4$, and $R^7$ has the meanings of $R^3$, the above radicals overall having a smaller number of halogen atoms than $R^1$, $R^4$ and $R^3$ or being halogen-free, is reacted with halogen, sulfuryl chloride and/or sulfuryl bromide.

2. A halogenated tertiary diisocyanate of the formula I

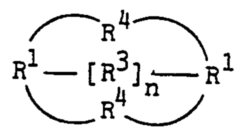

(I)

where n is 0 or 1 and the individual radicals $R^1$ are identical or different and, if n is 0, are

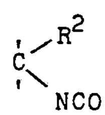

or

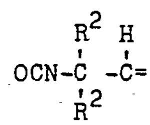

or, if n is 1, are

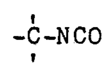

or

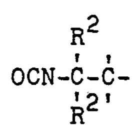

the individual radicals $R^2$ are identical or different and are aliphatic radicals, or two adjacent radicals $R^2$ linked by a carbon atom can be members of a halogen-free or halogenated alicyclic ring, and the individual radicals $R^3$ and $R^4$ can be identical or different and are aliphatic radicals.

**Revendications**

1. Procédé de préparation de diisocyanates tertiaires, halogénés, de la formule I

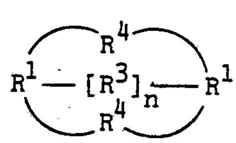

(I)

dans laquelle n est égal à 0 ou à 1 et les symboles individuels $R^1$ qui peuvent avoir des significations identiques ou différentes, représentent, lorsque n est égal à 0, le radical

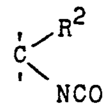

ou le radical

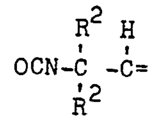

ou, lorsque n est égal à 1, le radical

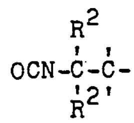

les symboles individuels $R^2$ qui peuvent avoir des significations identiques ou différentes représentent un radical aliphatique, deux symboles $R^2$ qui s'avoisinent par l'intermédiaire d'un atome de carbone pouvant également former des chaînons d'un noyau alicyclique exempt d'halogène ou halogéné, les symboles individuels $R^3$ et $R^4$ qui peuvent être identiques ou différents, représentent chacun un radical aliphatique, la substance finale contenant, au total, plus d'atomes d'halogène que la substance de départ II, par halogénation d'isocyanates, caractérisé en ce que l'on fait réagir des diisocyanates tertiaires de la formule II

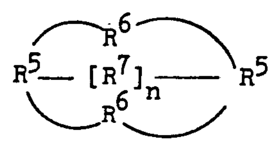 (II)

dans laquelle $R^5$ possède les mêmes significations que celles attribuées précédemment à $R^1$, $R^6$ possède les mêmes significations que celles attribuées à $R^4$ et $R^7$ possède les mêmes significations que celles attribuées à $R^3$, où les symboles précités sont, au total, plus pauvres en halogènes que les symboles $R^1$, $R^4$ et $R^3$ ou sont exempts d'halogènes, sur un halogène, le chlorure de sulfuryle et/ou le bromure de sulfuryle.

2. Diisocyanates tertiaires halogénés de la formule I

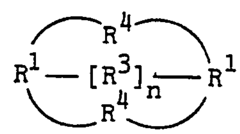 (I)

dans laquelle n est égal à 0 ou à 1 et les symboles individuels $R^1$ qui peuvent être identiques ou différents, représentent, lorsque n est égal à 0, le radical

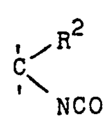

ou le radical

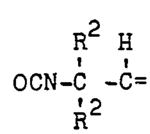

ou, lorsque n est égal à 1, le radical

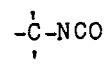

ou le radical

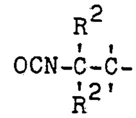

les symboles individuels $R^2$ qui peuvent être identiques ou différents, représentent un radical aliphatique, deux symboles $R^2$ voisins par l'intermédiaire d'un atome de carbone pouvant également former des chaînons d'un noyau alicyclique exempt d'halogène ou halogéné, les symboles individuels $R^3$ et $R^4$ qui peuvent être identiques ou différents, représentent chacun un radical aliphatique.